# EUROPÄISCHE PATENTSCHRIFT

(11) **EP 0 718 272 B1**
(45) Veröffentlichungstag und Bekanntmachung des Hinweises auf die Patenterteilung: **18.11.1998**
(21) Anmeldenummer: 95120040.1
(22) Anmeldetag: 19.12.1995
(51) Int. Cl.: C07C 67/343, C07C 69/63

(54) **Verfahren zur Herstellung von Trialkylester-Derivaten**
Process for the preparation of derivatives of trialkyle esters
Procédé de préparation de dérivés d'esters de trialkyle

(30) Priorität: 21.12.1994 CH 3868/94
(43) Veröffentlichungstag der Anmeldung: 26.06.1996
(73) Patentinhaber: LONZA AG, CH-3945 Gampel/Wallis (CH)
(72) Erfinder: Pfammatter, Elmar, Dr., DH-3930 Visp (Kanton Wallis) (CH)
(74) Vertreter: Weinhold, Peter, Dr.

(56) Entgegenhaltungen:
- JOURNAL OF ORGANIC CHEMISTRY, Bd. 44, Nr. 20, 28.September 1979 EASTON US, Seiten 3492-3496, HENRY C. PADGETT ET AL. 'THE ALKOXYCARBONYL MOIETY AS A BLOCKING GROUP. A GENERAL USEFUL VARIATION OF THE MALONIC ESTER SYNTHESIS'

## Beschreibung

Die Erfindung betrifft ein neues Verfahren zur Herstellung von Trialkylester-Derivaten der allgemeinen Formel 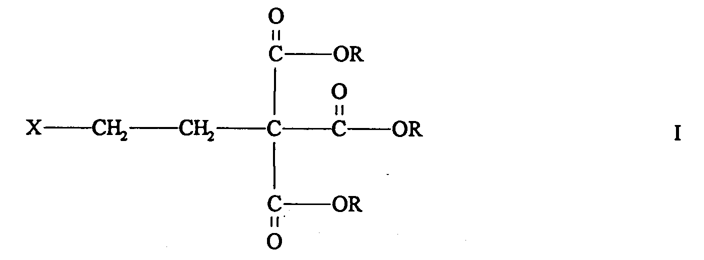 worin X Chlor, Brom, Jod, Tosyloxy- oder Mesyloxy- und R eine C₁―C₆-Alkylgruppe bedeutet.

Trialkylester-Derivate der Formel I wie z. B. Bromtriethylester sind wertvolle Zwischenprodukte zur Herstellung von antiviralen Nukleotidderivaten (EP-A 0 369 583).

Bekannt ist die Herstellung von Bromtriethylester ausgehend von Methantriethyltricarboxylat und Dibromethan in Gegenwart von Natriumethanolat (H. Rapoport et al., J. Org. Chem., Vol. 44, No. 20, 1979, 3492 - 3496).

Dieses Verfahren hat jedoch zum einen den Nachteil, dass das entsprechende Produkt stark mit Nebenprodukten verunreinigt ist, zum anderen, dass es aufgrund relativ langer Umsetzungszeiten unwirtschaftlich ist.

Aufgabe der vorliegenden Erfindung war ein wirtschaftlicheres Verfahren zur Herstellung von Trialkylester-Derivaten zur Verfügung zu stellen, wobei die entsprechenden Produkte in guter Reinheit erhalten werden.

Diese Aufgabe wird mit dem neuen Verfahren gemäss Anspruch 1 gelöst.

Erfindungsgemäss wird ein Methantrialkyltricarboxylat der allgemeinen Formel 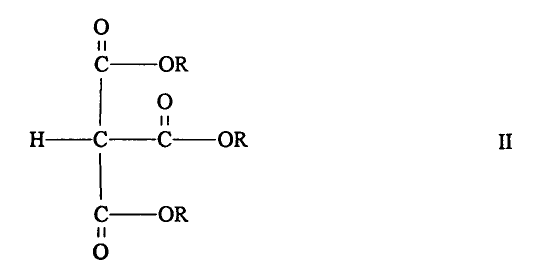 worin R die genannte Bedeutung hat, mit einem disubstituierten Ethan der allgemeinen Formel

X―CH₂―CH₂―X III

worin die beiden Substituenten X gleich oder verschieden sind und die genannte Bedeutung haben, in Gegenwart eines Erdalkali- oder Alkalicarbonats zum Endprodukt (Formel I) überführt.

Das als Edukt eingesetzte Methantrialkyltricarboxylat der allgemeinen Formel II kann auf bekannte Weise gemäss H. Lund & A. Voigt in Organic Syntheses, Collect. Vol. II, Wiley, New York, 1946, S. 594 hergestellt werden. Die als Edukte eingesetzten disubstituierten Ethane wie beispielsweise Dibromethan sind käuflich.

Als Methantrialkyltricarboxylat (Formel II) wird beispielsweise Methantrimethyl-, Methantriethyl-, Methantripropyl-, Methantriisopropyl-, Methantributyl-, Methantriisobutyl-, Methantripentyl, Methantriisopentyl-, Methantrihexyl- oder Methantriisohexyl-tricarboxylat angewendet. Vorzugsweise wird Methantriethyltricarboxylat eingesetzt.

Als disubstituierte Ethane (Formel III) können 1,2-Dibromethan, 1,2-Dichlorethan, 1,2-Dijodethan, 1-Brom-2-chlorethan, 1-Brom-2-jodethan, 1-Jod-2-chlorethan, 1-Brom-2-mesyloxyethan, 1-Chlor-2-mesyloxyethan, 1-Jod-2-mesyloxyethan, 1-Brom-2-tosyloxyethan, 1-Chlor-2-tosyloxyethan oder 1-Jod-2-tosyloxyethan verwendet werden. Vorzugsweise wird 1,2-Dibromethan verwendet.

Als Erdalkalicarbonat können Magnesium- oder Calciumcarbonat, als Alkalicarbonat Kalium-oder Natriumcarbonat angewendet werden. Vorzugsweise wird ein Alkalicarbonat wie Kalium-oder Natriumcarbonat, insbesondere Kaliumcarbonat, eingesetzt.

Zweckmässig wird die Umsetzung in einem polar aprotischen Lösungsmittel durchgeführt. Als polar aprotische Lösungsmittel sind beispielsweise Dimethylformamid, Dimethylacetamid und N-Methylpyrrolidon geeignet. Vorzugsweise wird die Umsetzung in Dimethylformamid durchgeführt.

Die Umsetzung wird zweckmässig bei einer Temperatur von 0 °C bis zur Rückflusstemperatur des entsprechenden Lösungsmittels, vorzugsweise von 20 bis 90 °C, durchgeführt.

Nach einer Umsetzungszeit von ca. 0,5 bis 5 h kann dann das Endprodukt gemäss Formel I durch fachmännisch übliche Aufarbeitungsmethoden erhalten werden. Im Unterschied zu dem bekannten Verfahren gemäss H. Rapoport et al., (J. Org. Chem., Vol. 44, No. 20, 1979, 3492 - 3496) sowie im Unterschied zu dem gemäss Beispiel 2 der vorliegenden Anmeldung durchgeführten Verfahren können nach dem erfindungsgemässen Verfahren die Endprodukte in einer wesentlich kürzeren Umsetzungszeit, in guter Reinheit und in hoher Ausbeute erhalten werden.

### Beispiele:

### Beispiel 1

### Herstellung von Bromtriethylester (mit K₂CO₃)

100,0 g (0,43 mol) Methantriethyltricarboxylat, 290 ml Dimethylformamid und 59,5 g (0,43 mol) K₂CO₃ wurden vorgelegt und innerhalb von 15 min. auf 60 °C erhitzt. Die weisse Suspension wurde weitere 15 min. bei dieser Temperatur gerührt und dann während einer Stunde und 40 min. bei 60 °C mit 120 g (0,63 mol) Dibromethan versetzt. Danach wurde 20 min. bei 60 °C nachgerührt.

Zur Aufarbeitung wurde die Suspension im Eisbad abgekühlt, und man gab dann 500 ml Wasser zu. Die organische Phase wurde mit 240 ml Toluol extrahiert. Die vereinigten organischen Phasen wurden mit 200 ml Wasser versetzt (pH = 8,6), das Zweiphasengemisch wurde gut durchmischt und mit 11,06 g 10%iger NaOH-Lösung auf einen pH von 12,3 eingestellt. Nach 10 min. Rühren wurden die Phasen getrennt.
Das Lösungsmittel wurde in einem Luwadurchgang abdestilliert. (Bedingungen: Wasserstrahlvakuum, Öltemperatur 118 °C, Drehzahl 1250 U/min., Speisung 4 - 5 ml/min.)
126,2 g Bromtriethylester (Gehalt 94%, Fl%) wurden erhalten.
Die Bedingungen für den 2. Durchgang waren: Vakuum 5 mbar, Öltemperatur 197 °C, 1250 U/min., Speisung 2 - 3 ml/min.
Insgesamt wurden 116,2 g Bromtriethylester erhalten (Gehalt 98,7 %, Fl%) entsprechend einer Ausbeute von 80%, bezogen auf eingesetztes Methantriethyltricarboxylat.

### Beispiel 2:

### Herstellung von Bromtriethylester (mit Natriumethanolat; Vergleichsbeispiel)

100 g (0,43 mol) Methantriethyltricarboxylat wurden in 290 ml Dimethylformamid gelöst und mit 30,9 g (0,42 mol) Natriumethanolat versetzt. Die Temperatur stieg auf 60 °C. Danach wurden unter Wasserstrahlvakuum bei einer Blasentemperatur von 50 °C 55 ml Dimethylformamid / Ethanol-Gemisch während 45 min. abdestilliert. 55 ml Dimethylformamid wurden wieder zugegeben. Die Reaktionsmischung wurde auf 75 - 80 °C erwärmt und noch während dem Aufwärmen mit 157 g (0,83 mol) Dibromethan während 90 min. versetzt. Die gebildete Suspension wurde 3 h bei 80 °C nachgerührt und wie folgt aufgearbeitet.

Dazu wurde die Suspension bei Raumtemperatur mit 240 ml E-Wasser und 240 ml Toluol versetzt (Temperatur stieg auf 40 °C). Mit 18,5 g 10%iger wässriger NaOH-Lösung wurde der pH des Zweiphasengemisches von 7 auf 12,5 eingestellt; diese Mischung rührte man 15 min., danach wurden die Phasen getrennt. Die basische Wasserphase wurde mit 120 ml Toluol extrahiert (ca. 12% Bromtriethylester wurden so noch erhalten). Die organischen Phasen wurden vereinigt (Rohausbeute 94,4%).

Durch Destillation im Dünnschichtverdampfer wurden (Wasservakuum, 110 °C Öltemperatur, 1250 U/min., 4 - 5 ml/min.) 131 g Rohbromtriethylester (Gehalt: 92,5%; 1,3% Bromtriethylester, waren im Destillat) erhalten. Ein zweiter Destillationsdurchgang im Dünnschichtverdampfer ergab 120 g Bromtriethylester (Gehalt 94,8%), entsprechend einer Ausbeute von 82%, bezogen auf Methantriethyltricarboxylat.
Im Gegensatz zu Beispiel 1 wurde nach diesem Verfahren Bromtriethylester in einer schlechteren Qualität erhalten, da dieser zu mindestens 4% mehr verunreinigt war.

## Patentansprüche

1. Verfahren zur Herstellung von Trialkylester-Derivaten der allgemeinen Formel 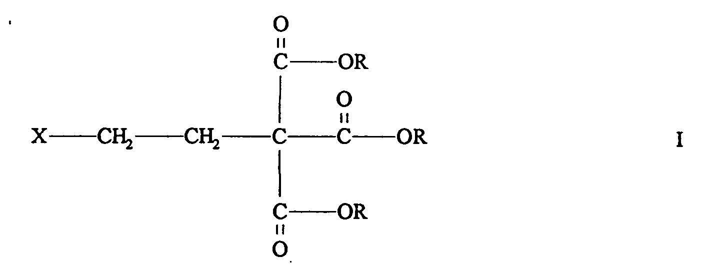 worin X Chlor, Brom, Jod, Tosyloxy- oder Mesyloxy- und R eine C₁―C₆-Alkylgruppe bedeutet, dadurch gekennzeichnet, dass man ein Methantrialkyltricarboxylat der allgemeinen Formel 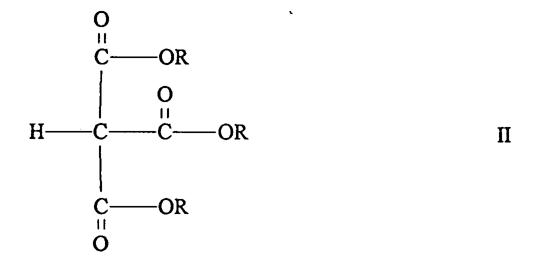 worin R die genannte Bedeutung hat, mit einem disubstituierten Ethan der allgemeinen Formel
X―CH₂―CH₂―X III
worin die beiden Substituenten X gleich oder verschieden sind und die oben genannte Bedeutung haben, in Gegenwart eines Erdalkali- oder Alkalicarbonats zum Endprodukt gemäss Formel I überführt.

2. Verfahren nach Patentanspruch 1, dadurch gekennzeichnet, dass man die Umsetzung in einem polar aprotischen Lösungsmittel durchführt.

3. Verfahren nach Patentanspruch 2, dadurch gekennzeichnet, dass man als polar aprotisches Lösungsmittel Dimethylformamid anwendet.

4. Verfahren nach mindestens einem der Ansprüche 1 bis 3, dadurch gekennzeichnet, dass man die Umsetzung in Gegenwart eines Alkalicarbonats durchführt.

5. Verfahren nach Patentanspruch 4, dadurch gekennzeichnet, dass man als Alkalicarbonat Kalium- oder Natriumcarbonat anwendet.

6. Verfahren nach mindestens einem der Ansprüche 1 bis 5, dadurch gekennzeichnet, dass man als disubstituiertes Ethan 1,2-Dibromethan anwendet.

7. Verfahren nach mindestens einem der Ansprüche 1 bis 6, dadurch gekennzeichnet, dass man als Methantrialkyltricarboxylat Methantriethyltricarboxylat anwendet.

8. Verfahren nach mindestens einem der Ansprüche 1 bis 7, dadurch gekennzeichnet, dass man die Umsetzung bei einer Temperatur von 0 °C bis zur Rückflusstemperatur des entsprechenden Lösungsmittels durchführt.

## Claims

1. A method for producing trialkyl ester derivatives having the general formula 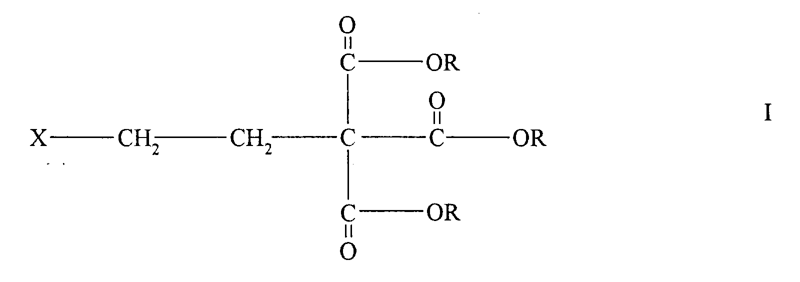 wherein X designates chlorine, bromine, iodine, tosyloxy- or mesyloxy-, and R designates a C₁-C₆ alkyl group, characterised in that a methanetrialkyltricarboxylate having the general formula 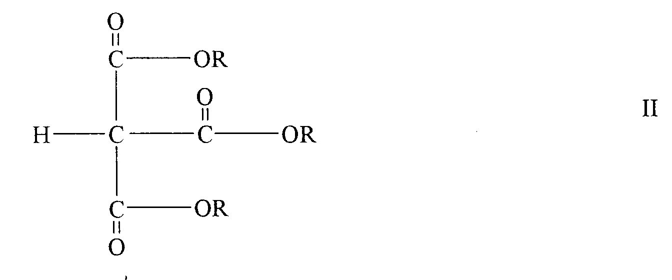 wherein R has the above meaning, is reacted with a disubstituted ethane having the general formula
X―CH₂―CH₂―X III
wherein the two substituents X are the same or different and have the above meaning, in the presence of an alkaline earth carbonate or alkali carbonate to form the final product of Formula I.

2. The method according to claim 1, characterised in that reaction is carried out in a polar aprotic solvent.

3. The method according to claim 2, characterised in that dimethylformamide is used as a polar aprotic solvent.

4. The method according to at least one of claims 1 to 3, characterised in that reaction is carried out in the presence of an alkali carbonate.

5. The method according to claim 4, characterised in that potassium carbonate or sodium carbonate is used as an alkali carbonate.

6. The method according to at least one of claims 1 to 5, characterised in that 1,2-dibromoethane is used as a disubstituted ethane.

7. The method according to at least one of claims 1 to 6, characterised in that methanetriethyltricarboxylate is used as a methanetrialkyltricarboxylate.

8. The method according to at least one of claims 1 to 7, characterised in that reaction is carried out at a temperature from 0°C up to the reflux temperature of the corresponding solvent.

## Revendications

1. Procédé pour la préparation de dérivés de trialkylesters de la, formule générale 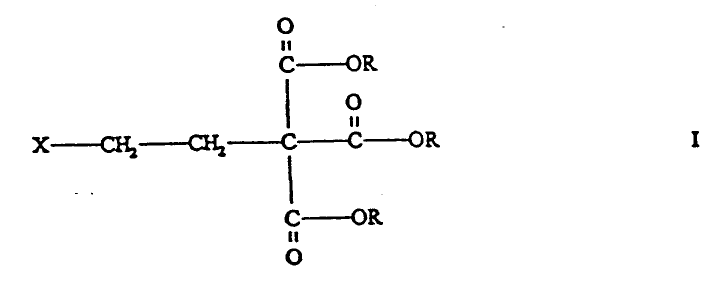 dans laquelle X signifie le chlore, le brome, l'iode, un groupe tosyloxy ou mésyloxy et R un groupe alkyle C₁-C₆, caractérisé en ce que l'on transforme en le produit final selon la formule I un trialkyltricarboxylate de méthane de la formule générale 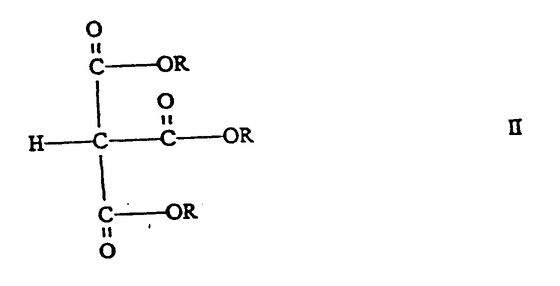 dans laquelle R a la signification citée avec un éthane disubstitué de la formule générale
X―CH₂―CH₂―X III
dans laquelle les deux substituants X sont identiques ou différents et ont la signification ci-dessus, en présence d'un carbonate alcalin ou alcalino-terreux pour obtenir le produit final de la formule I.

2. Procédé selon la revendication 1, caractérisé en ce que l'on conduit la réaction dans un solvant aprotique polaire.

3. Procédé selon la revendication 2, caractérisé en ce qu'en tant que solvant aprotique polaire, on utilise du diméthylformamide.

4. Procédé selon au moins l'une des revendications 1 à 3, caractérisé en ce que l'on conduit la réaction en présence d'un carbonate alcalin.

5. Procédé selon la revendication 4, caractérisé en ce qu'en tant que carbonate alcalin, on utilise du carbonate de potassium ou de calcium.

6. Procédé selon au moins l'une des revendications 1 à 5, caractérisé en ce qu'en tant qu'éthane disubstitué, on utilise du 1,2-dibrométhane.

7. Procédé selon au moins l'une des revendications 1 à 6, caractérisé en ce qu'en tant que trialkyltricarboxylate de méthane, on utilise du triéthyltricarboxylate de méthane.

8. Procédé selon au moins l'une des revendications 1 à 7, caractérisé en ce que l'on conduit la réaction à une température de 0°C jusqu'à la température de reflux du solvant correspondant.
